# EUROPÄISCHE PATENTSCHRIFT

(11) **EP 0 630 892 B1**
(45) Veröffentlichungstag und Bekanntmachung des Hinweises auf die Patenterteilung: **17.11.1999**
(21) Anmeldenummer: 94107420.5
(22) Anmeldetag: 13.05.1994
(51) Int. Cl.: C07D 309/10, C09K 19/34, C09K 19/58

(54) **Chirale Verbindungen**
Chiral compounds
Composés chiraux

(30) Priorität: 19.05.1993 DE 4316826; 12.03.1994 DE 4408414; 12.03.1994 DE 4408413
(43) Veröffentlichungstag der Anmeldung: 28.12.1994
(73) Patentinhaber: BASF Aktiengesellschaft, 67063 Ludwigshafen (DE)
(72) Erfinder: Delavier, Paul, Dr., D-67061 Ludwigshafen (DE); Siemensmeyer, Karl, Dr., D-67227 Frankenthal (DE); Vill, Volkmar, Dr., D-20255 Hamburg (DE); Hartwig, Angela, D-22926 Ahrensburg (DE); Gesekus, Gunnar, D-22111 Hamburg (DE); Tunger, Hanns-Walter, D-22085 Hamburg (DE)

(56) Entgegenhaltungen:
- EP-A- 0 405 983
- EP-A- 0 548 947
- WO-A-93/13093
- PATENT ABSTRACTS OF JAPAN vol. 12, no. 92 (C-483) 25. März 1988 & JP-A-62 226 974 (MITSUI PETROCHEM IND) 5. Oktober 1987
- Beilstein Handbook of Organic Chemistry, 4th Edition, Centennial Index, General Compound Name Index for the Basic Series and Supplementary Series I, II, III and IV, Volume 28, Part 9

## Beschreibung

Chirale, smektisch flüssigkristalline Materialien, die beim Abkühlen aus der flüssigkristallinen Phase glasartig unter Ausbildung einer Schichtstruktur erstarren, werden bekanntermaßen auf elektrooptischem Gebiet für viele Zwecke eingesetzt. Zu nennen sind hier beispielsweise optische Speichersysteme (DE-A-38 27 603 und DE-A-39 17 196), die Elektrophotografie (DE-A-39 30 667), flüssigkristalline Anzeigeelemente wie Displays (Mol. Cryst. Liq. Cryst., 114, 151 (1990)) sowie bei gleichzeitig vorliegendem ferroelektrischem Verhalten elektrische Speichersysteme (Ferroelectrics, 104, 241(1990)).

In der Schichtstruktur ferroelektrischer S_{c}*-Phasen sind die Moleküllängsachsen innerhalb der einzelnen Schicht gegenüber der Schichtnormalen z geneigt. Die Richtung dieser Neigung wird durch den Direktor n angegeben, der Winkel zwischen z und n ist der sogenannte Tiltwinkel Θ. S_{c}*-Phasen weisen zwei stabile Zustände mit unterschiedlicher Richtung von n auf, zwischen denen durch Anlegen eines elektrischen Feldes geschaltet werden kann (elektrooptischer Effekt).

S_{c}*-Phasen treten bei niedermolekularen, flüssigkristallinen Materialien, bei Oligomesogenen und bei polymer ferrolelektrischen Materialien auf, wobei die wesentlichen Eigenschaften der S_{c}*-Phasen übereinstimmen.

Die bislang hergestellten flüssigkristallinen Materialien weisen jedoch Nachteile auf, zum Beispiel geringe spontane Polarisation, geringe Phasenbreite, kein stabiles, getiltet smektisches Glas bei Raumtemperatur oder zu langsames Schalten.

Das Auftreten der flüssigkristallinen S_{c}*-Phase wird durch alle Gruppen des Moleküls, d.h. Spacer A, mesogene Gruppe M und die chirale Gruppe in erheblichem Ausmaß beeinflußt, sodaß kleinste Änderungen der molekularen Struktur S_{c}*-Phasen induzieren oder auch zum Verschwinden bringen können.

Speziell die chirale Gruppe ist durch ihre Struktur und spezielle Funktion für das Zustandekommen einer spontanen Polarisation von entscheidender Bedeutung.

Der vorliegenden Erfindung liegt die Aufgabe zugrunde, chirale Gruppen für flüssigkristalline Materialien zu suchen, die die flüssigkristallinen Eigenschaften möglichst wenig stören, gleichzeitig hohe spontane Polarisationen induzieren und synthetisch verfügbar sind.

Die Erfindung betrifft daher Verbindungen der Formel I 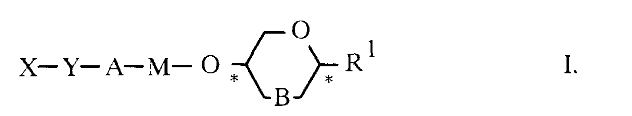 in der bedeuten
- R¹: gegebenenfalls durch Fluor, Chlor, Brom, Cyan oder Hydroxy substituiertes und gegebenenfalls durch O, S, NH, N(CH₃), COO oder OCO unterbrochenes C₁- bis C₃₀-Alkyl, gegebenenfalls durch Fluor, Chlor, Brom, Cyan, C₁- bis C₂₀-Alkyl, C₁- bis C₂₀-Alkoxy oder C₁- bis C₂₀-Alkoxycarbonyl substituiertes Phenyl oder Fluor, Chlor, Brom oder Cyan,
- B: eine Einfach- oder Doppelbindung,
- X: eine polymerisierbare Gruppe oder Wasserstoff oder Hydroxy,
- Y: eine direkte Bindung oder O, OCO, COO oder S,
- A: ein gegebenenfalls durch Fluor, Chlor, Brom, Cyan oder Hydroxy substituierter C₁- bis C₃₀-Alkylrest, bei dem gegebenenfalls jedes dritte C-Atom durch O, S, NH, N(CH₃), OCO oder COO ersetzt sein kann, und
- M: eine mesogene Gruppe der Formel 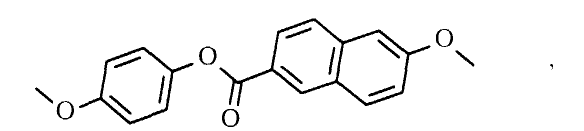 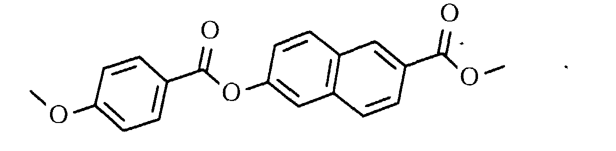 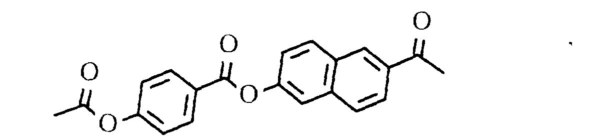 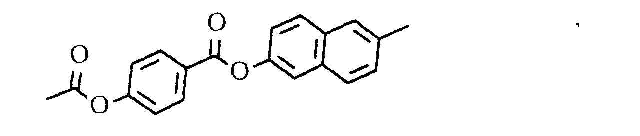 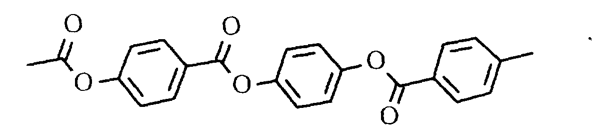 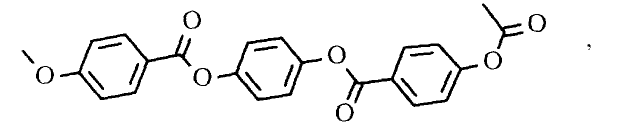 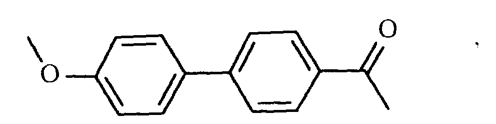 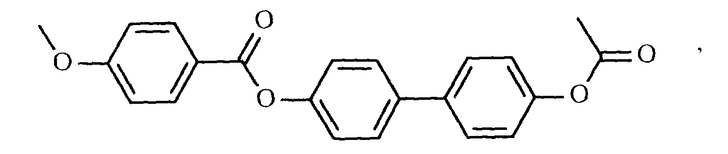 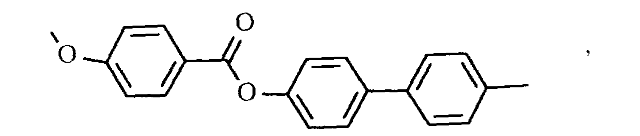 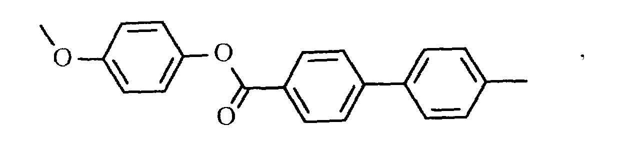 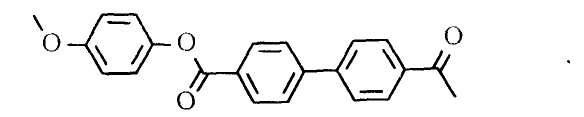 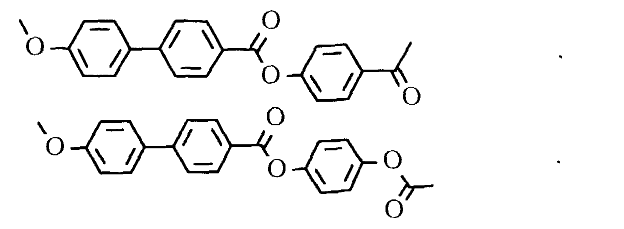 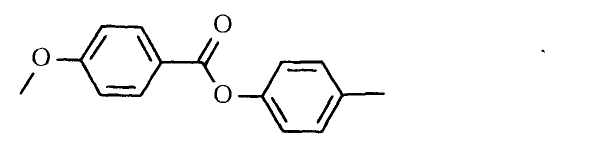 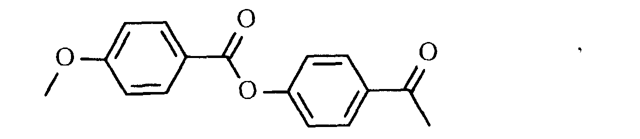 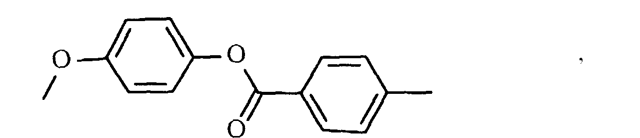 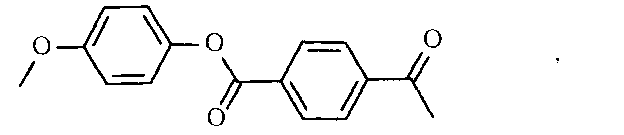 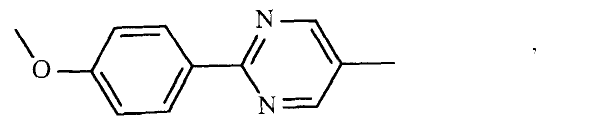 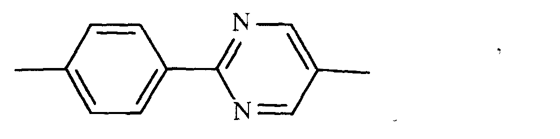 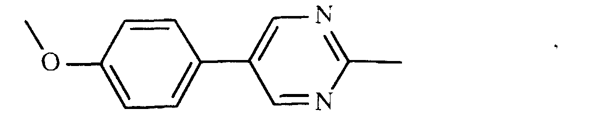 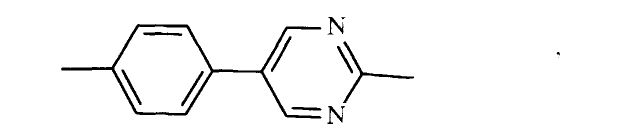 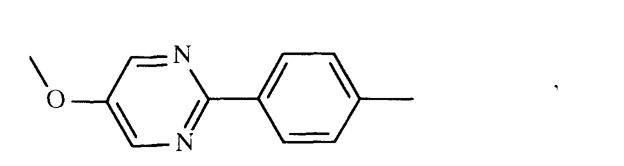 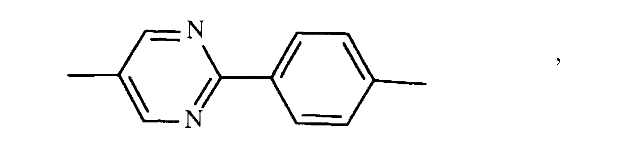 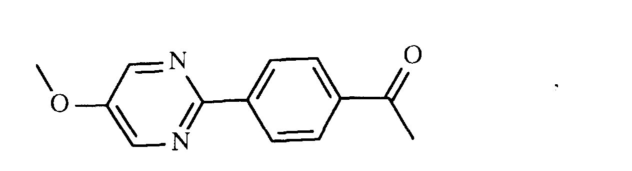 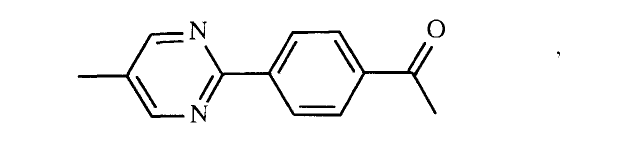 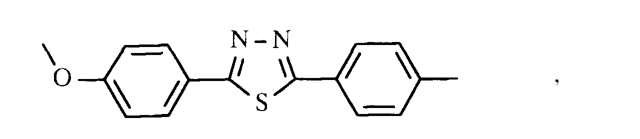 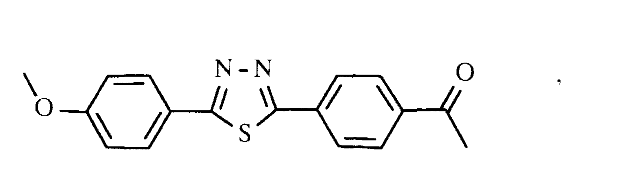 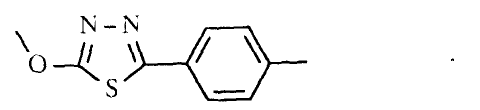 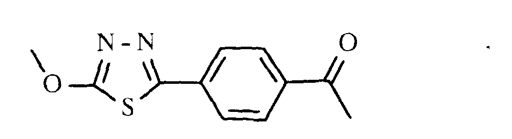 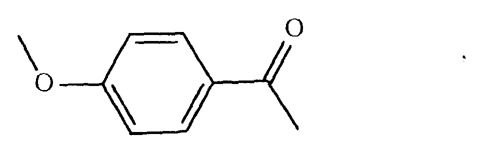 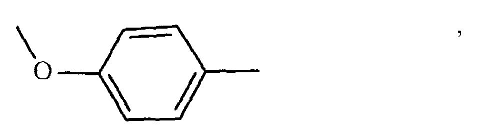 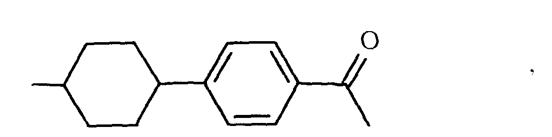 oder 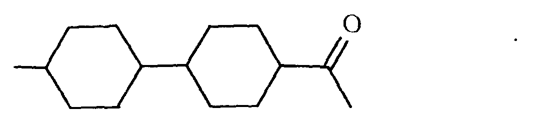 Weiterhin betrifft die Erfindung die Herstellung von Polymeren und Oligomesogenen aus den Monomeren der allgemeinen Formel I, wenn X 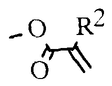 oder Hydroxy ist, wobei R² Methyl, Chlor, Brom, CN oder vorzugsweise Wasserstoff bedeutet, sowie deren Verwendung zum Aufbau von Aufzeichnungsschichten für laseroptische und elektrische Aufzeichnungselemente, in der Elektrophotografie, zu- Erzeugung latenter Ladungsbilder, zum Aufbau oder als Bestandteil von flüssigkristallinen Anzeigeelementen sowie als farbige Reflektoren.

Beispiele für den Rest der Formel 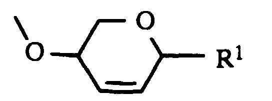 sind folgende Gruppen: 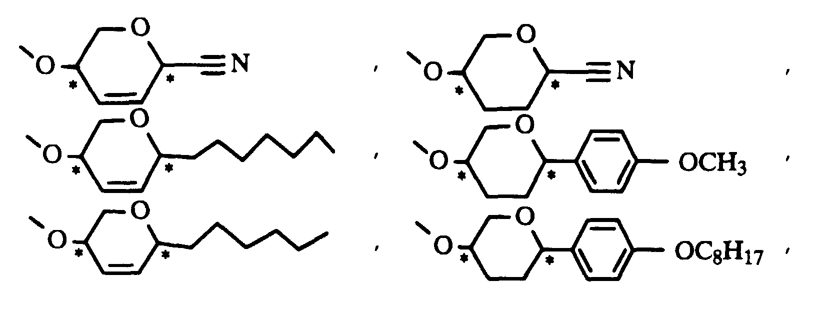 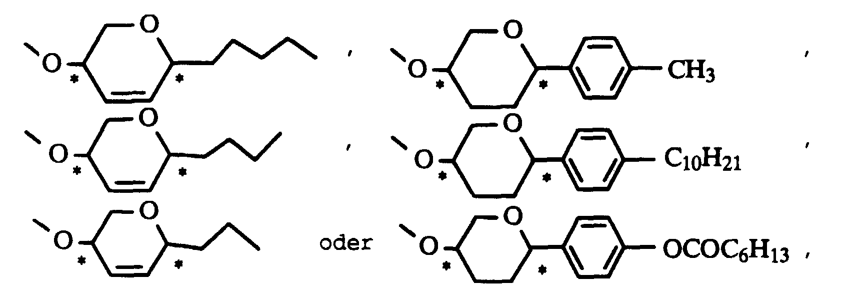 sowie die gleichen Reste mit einer Einfachbindung anstelle der Doppelbindung.

Die Herstellung der erfindungsgemäßen Verbindungen erfolgt nach an sich bekannten Methoden (s. z.B. DE-A-39 17 186 oder Mol. Cryst. Liq. Cryst-. 191, 231 (1991)), im folgenden sei sie anhand eines verallgemeinerungsfähigen Beispiels erläutert.

Die Phasenumwandlungstemperaturen wurden mit einem Leitz Polarisationsmikroskop (Ortholux II pol) in Verbindung mit einem Mettler Mikroskopheiztisch (Mettler FP 800/84) bestimmt.

Folgende Abkürzungen werden für die verschiedenen Phasen verwendet:
- K, K₁, K₂:: unterschiedliche kristalline Phasen
- I:: Isotrope Phase
- ch:: cholesterische Phase

### Beispiel 1

Herstellung von (2S,5S)-5-(4'-Butoxyphenyl)-carboxyl-2-cyano-2H-5,6-dihydropyran 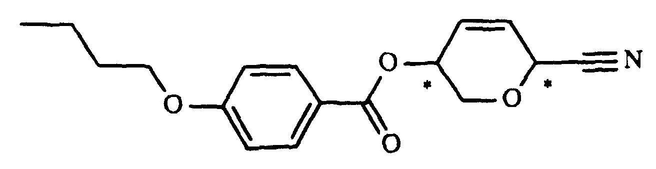
a) Herstellung von 3,4-Di-O-acetyl-D-xylal
   60 g (188 mmol) D-Xylosetetraacetat werden in 22ml Essigsäureanhydrid und 22 ml Essigsäure gelöst. Nach dem Abkühlen auf 0°C gibt man tropfenweise 130 ml Bromwasserstoff/Eisessig (33%ig) hinzu und läßt bei Raumtemperatur 2 h rühren. Die rohe Acetobromxylose-Lösung wird im Laufe von 30 min bei 0°C zu einer Reduktionsmischung aus 90 g Natriumacetat-Trihydrat, 150 ml Wasser, 150 ml Essigsäure, 300 ml Aceton und 250g Zink zugetropft. Die Temperatur sollte dabei unter 10°C gehalten werden. Nach einer Reaktionszeit von 180 min ist die Reaktion beendet. Nachdem man das Zink abfiltriert hat, wäscht man den Filterrückstand mit einem Essigsäure/Wasser-Gemisch (1:1). Das entstandene 3,4-Di-O-acetyl-D-xylal wird aus dem Filtrat durch dreimaliges Ausschütteln mit Chloroform extrahiert und der Extrakt dann mit gesättigter Natriumhydrogencarbonatlösung neutralisiert. Die Reinigung des Produktes erfolgt säulenchromatografisch (Kieselgel 60, Petrolether/Diethylether 3/1).
   Ausbeute: 21 g (=60% d. Theorie)
   [α]²⁰_{D} = -87.8 (c=1, Chloroform)
   Schmelzpunkt: 37°C
b) Herstellung von (2S,5S)-5-Hydroxy-2-cyano-2H-5,6-dihydropyran
   200 mg 3,4-Di-O-acetyl-D-xylal und 100 mg Trimethylsilylcyanid in 10 ml absolutem Acetonitril werden mit 1 Tropfen Bortrifuoridetherat versetzt. Nach 10 min wird die Lösung mit festem Natriumhydrogencarbonat neutralisiert, das Lösungsmittel abgedampft und die Substanz säulenchromatografisch (Essigester/Toluol = 1/4) gereinigt.
   Anschließend wird die Substanz in methanolischer Ammoniaklösung aufgenommen und über Nacht stehengelassen. Anschließend wird das Lösungsmittel abgedampft.
   Ausbeute: 120 mg
   [α]²⁰_{D} = +271.0 (c=1, Chloroform)
c) Herstellung von (2S,5S)-5-(4'-Butoxyphenyl)-carboxyl-2-cyano-2H-5,6-dihydropyran
   30 mg 4'-Butyloxyphenylcarbonsäure werden in der fünffachen Menge Thionylchlorid gelöst und mit einem Tropfen Dimethylformamid versetzt. Es wird bei Raumtemperatur gerührt, bis eine klare Lösung entstanden ist. Anschließend wird noch für 10 min auf 40°C erwärmt und dann das Lösungsmittel abdestilliert. Das Säurechlorid wird mehrere Stunden unter Ölpumpenvakuum getrocknet.
   20 mg (2S,5S)-5-Hydroxy-2-cyano-2H-5,6-dihydropyran und 12 mg 4-Dimethylaminopyridin werden in der fünffachen Gewichtsmenge absolutem Pyridin gelöst und zu einer Lösung von 25 mg des Säurechlorides in absolutem Dichlormethan gegeben. Die Mischung rührt über Nacht bei Raumtemperatur und anschließend eine halbe Stunde bei 40°C. Dann wird das Lösungsmittel abdestilliert, dreimal in Toluol aufgenommen und wieder zur Trockne eingedampft. Die Reinigung erfolgt säulenchromatografisch mit Dichlormethan als Laufmittel.
   Ausbeute: 19.2 mg
   [α]²⁰_{D} = +125.7 (c=1, Chloroform)
   Schmelpunkt: 91.7°C

Analog Beispiel 1 wurden die Verbindungen der Beispiele 2 bis 9 hergestellt:

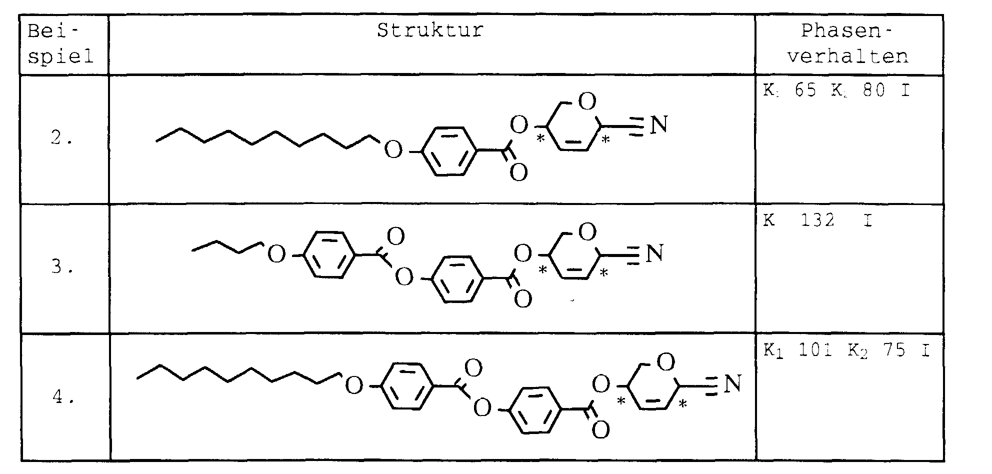

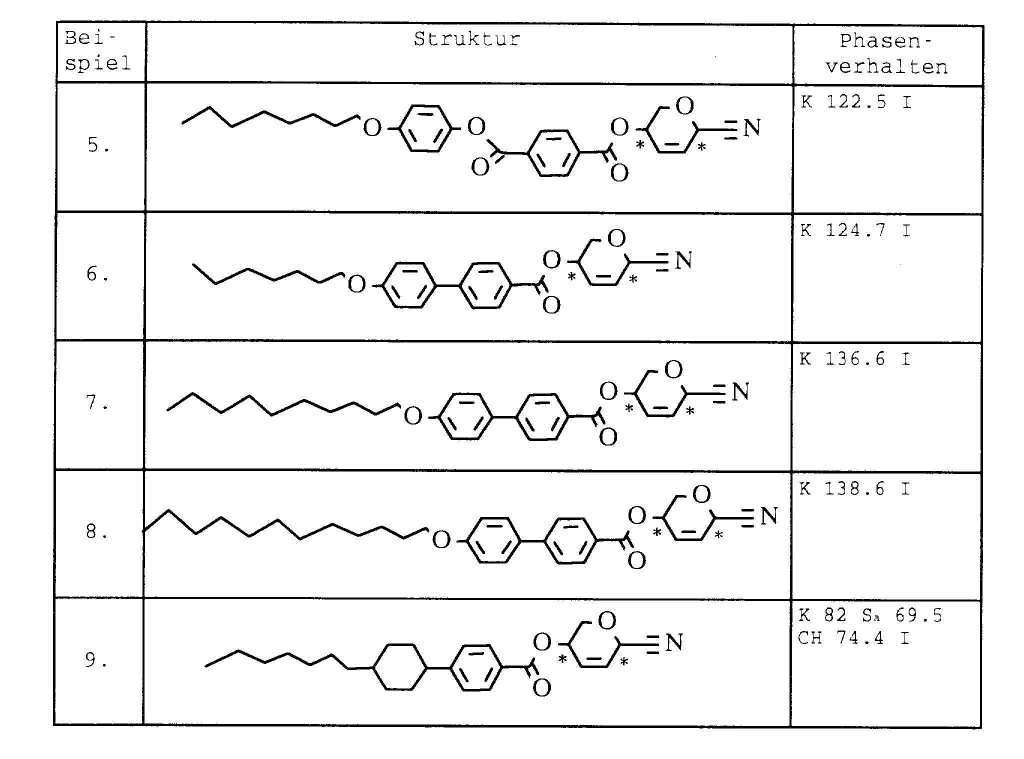

### Beispiel 10 (nicht beansprucht)

a) Darstellung der C-Glycoside
   Zu einer Lösung von 2 g (10 mmol) Diacetylxylal in 20 ml Dichlorethan werden bei Raumtemperatur 10 mmol des nucleophielen Reagenz (Trimethylsilylpropin) gegeben. Nach 5 min Rühren werden 0,1 ml Zinntetrachlorid langsam dazugegeben. Nach einer Reaktionszeit von 120 min gibt man 300 mg Kaliumcarbonat hinzu und rührt weitere 30 min. Nach Filtration wird zur Trockene einrotiert und das entstandene Produkt säulenchromatographisch (Petrolether/Essigester, 4:1) aufgetrennt.
   A-O-Acetyl-1-propinyl-2,3-didesoxy-β-D-glyceropento-2-enopyranosid
   Ausbeute: 1,45 g (80) %, gelber Sirup, [α]_{D}²⁰ = +237,3 (c = 1, Chloroform) ¹H-NMR (400 MHz, CDCl₃)
   8 = 5,8 ppm (dd, 1H, H-2), 5,73 (m, 1H, H-3), 4,83 (m, 1H, H-4), 4,74 (m, 1H, H-1), 3,95 (dd, 1H, H-5eq), 3,67 (td, 1H, H-5ax), 2,10 (s, 3H, Acyl-CH₃), 1,88 (s, 3H, H-CH₃)
   J_{1,2} = 3,0,J,₂₃ = 10,2,J_{3,4} = 5,1 J_{4,5eq} = 3,1,J_{4,5ax} =
   2,0,J_{5ax,5eq} = 12,8 Hz 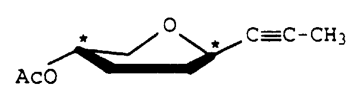
b) Analog Beispiel 10a) wurde hergestellt:
   4-O-Acetyl-1-pentinyl-2,3-dodesoxy-β-D-glyceropento-2-enopyranosid
   Ausbeute: 1,9 g (91) %, gelber Sirup, [α]_{D}²⁰ = +253,2 (c = 1, Chloroform) ¹H-NMR (400 MHz, CDCl₃)
   δ = 5,82 ppm (dd, 1H, H-2), 5,74 (m, 1H, H-3), 4,85 (m, 1H, H-4), , 4,76 (m, 1H, H-1), 4,02 (dd, 1H, H-5eq), 3,69 (td, 1H, H-5ax) , 2,03 (dt, 2H, H-CH₃), 1,90 (s, 3H, Acyl-CH₃), 1,37 (m, 2H, H-CH₂), 0,81 (s, 3H, H-CH₃)
   J_{1,2} = 3,0,J,₂₃ = 10,0,J_{3,4} = 5,0 J_{4,5eq} = 3,0,J₅ₐₓ,_{5eq} = 12,8 J_{CH2CH3} = 7,1 Hz 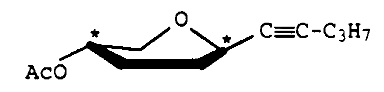
c) Analog Beispiel 10a) wurde hergestellt:
   4-Acetyl-1-hexinyl-2,3-didesoxy-β-d-glyceropento-2-enopyranosid
   Ausbeute: 1,75 g (79 %), gelber Sirup, [α]_{D}²⁰ = +168,3 (c = 1, Chloroform)
   δ = 5,82 ppm (dd, 1H, H-2), 5,74 (m, 1H, H-3), 4,83 (m, 1H, H-a), 4,00 (dd, 1H, H-5eq), 3,68 (td, 1H, H-5ax), 2,01 (dt, 2H, H-CH₂), 1,90 (s, 3H, Acyl-CH₃), 1,36 (m, 2H, H-CH₂), 1,25 (m, 2H, H-CH₂), 0,80 (s, 3H, H-CH₃)
   H_{1,2} = 3,5,J,₂₃ = 10,0.J_{3,4} = 5,0 J_{4,5eq} = 3,0,J_{5ax,5eq} = 13,0,J_{CH2,CH3} = 7,1 Hz

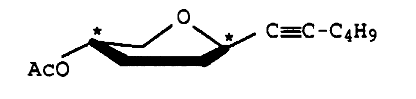

### Beispiel 11 (nicht beansprucht)

a) Hydrierung des Produktes aus Beispiel 10a):
   500 mg (3 mmol) werden in 50 ml Essigester/Ethanol (3:1) gelöst und mit Palladium/Kohle (10 %ig) als Katalysator unter Normaldruck hydriert. Nach 6 h ist die Reaktion beendet. Nach Abfiltrieren des Katalysators wird im Vakuum eingeengt. Das entstandene Produkt wird säulenchromatographisch (Petrolether/Essigester, 4:1) aufgetrennt.
   4-O-Acetyl-1-propanyl-2,3,5-tridesoxy-β-D-glyceropentopyranosid
   Ausbeute: 378 mg (78 %), gelber Sirup, [α]_{D}²⁰ = +64,6 (c = 1,
   Chloroform) ¹H-NMR (400 MHz, CDCl₃)
   δ = 4,38 ppm (m, 1H, H-4), 4,08 (ddd, 1H, H-a), 3,82 (t, 2H, H-5ₐₓ,5_{eq}), 2,29 (m, 1H-H-2_{eq}), 2,10 (s, 3H, Acyl-CH₃), 1,89 (m, 1-H, H-3_{eq}), 1,54-1,44 (m, 6H, H-2ₐₓ,3ₐₓ, dCH₂, bCH₂), 0,91 (s, 3H, H-CH₃)
   J_{1,2ax} = 10,51,H_{1,2eq} = 5,0,J_{5ax,5eq} = 10,1,_{2eq,3eq ∼}
   0,J_{2eq,2ax=2eq,3ax} = 13,0), J_{4,3eq} = 5,1,J_{4,5ax} = 10,5,J_{4,5eq} = 5,0,J_{CH2,Ch3} = 6,6 HZ. 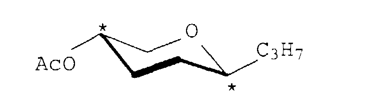
b) Analog Beispiel 11a) wurde das Produkt von Beispiel 10b) hydriert:
   Das NMR-Spektrum entsprechend dem des Produktes aus Beispiel 4 mit den in Bereich 1,54-1,44 ppm auftretenden NMR-Signalen für die zusätzlichen -CH₂-Gruppen.
   Ausbeute: 75 % 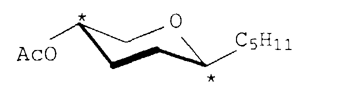
c) Hydrierung des Produktes aus Beispiel 10c) analog Beispiel 11a):
   Das NMR-Spektrum entsprechend dem des Produktes aus Beispiel 4 mit den in Beispiel 1,54-1,44 ppm auftretenden NMR-Signalen für die zusätzlichen -CH₂-Gruppen. Ausbeute: 68 %

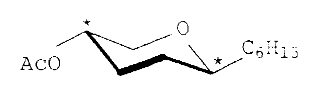

### Beispiel 12 (nicht beansprucht)

a) Herstellung von 4-Hydroxyl-1-propanyl-2,3,5-tridesoxy-b-D-glyceropento-pyranosid:
   1 mmol des Esters 4,5 oder 6 wird in 5 ml absolutem Methanol gelöst und bis zur basischen Reaktion mit 0,1 N methanolischer Natriummethanolat-Lösung tropfenweise versetzt und 6 h bei Raumtemperatur gerührt. Anschließend wird mit Kohlendioxid (fest) neutralisiert und abfiltriert. Die Reaktionskontrolle erfolgt mittels Dünnschichtchromatographie (Chlorophorm/Methanol 9:1). Auf eine Reinigung der Produkte wurde verzichtet. Ausbeute: 95 % 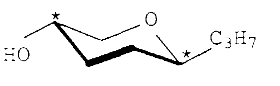
b) Herstellung von 4-Hydroxyl-1-pentanyl-2,3,5-tridesoxy-b-D-glyceropento-pyranosid
   Analog Beispiel 12a) wurde 4-Hydroxyl-1-pentanyl-2,3,5-tridesoxy-b-D-glyceropento-pyranosid ausgehend von Beispiel 11b) hergestellt.
   Ausbeute: 94 % 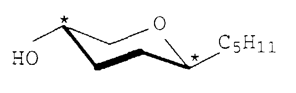
c) Herstellung von 4-Hydroxyl-1-lhexanyl-2,3,5-tridesoxy-b-D-glyceropento-pyranosid
   Analog Beispiel 12a) wurde 4-Hydroxyl-1-lhexanyl-2,3,5-tridesoxy-b-D-glyceropento-pyranosid ausgehend von Beispiel 11c) hergestellt.
   Ausbeute: 96 %

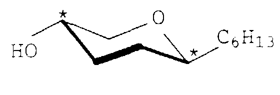

### Beispiel 13

### Synthese der Verbindung C₁₂H₁₅O-Ph-Ph-CO₂-THP-(CH₂)₂-CH₃

Ein Äquivalent 4-Dodecyloxy-4'-carboxy-biphenyl wird mit der fünffachen Gewichtsmenge Thionylchlorid und einem Tropfen Dimethylformamid versetzt. Es wird bei Raumtemperatur gerührt, bis eine klare Lösung entstanden ist. Anschließend wird noch für 10 min auf 40°C erwärmt und dann eingedampft. Das Säurechlorid wird mehrere Stunden am Ölpumpenvakuum getrocknet. Das Säurechlorid, gelöst in Dichlormethan, wird zu einer Lösung der Verbindung aus Beispiel 12a) in der fünffachen Menge absolutem Pyridin gegeben. Die Mischung rührt über Nacht bei Raumtemperatur, dann noch 30 min bei 40°C. Anschließend wird das Pyridin mit Toluol codestilliert. Die Produkte werden saulenchromatographisch gereinigt (Dichlormethan).
Ausbeute: 75 %

Analog Beispiel 13 wurden die Verbindungen der Beispiele 14 bis 19 hergestellt. Die Ausbeuten betragen durchweg mehr als 70 %.

Die Phasenumwandlungstemperaturen der Beispiele 14 bis 19 sind in der Tabelle angegeben.

| | Produkte | Umwandlungstemperaturen |
|---|---|---|
| Beispiel 14 | C₈H₁₇-Py-Ph-COO-THP-(CH₂)₂CH₃ | K 70,9 S_{A} 67,8 Ch 85,1 I |
| Beispiel 15 | C₁₀H₂₁O-Ph-Ph-COO-THP-(CH₂)₄CH₃ | K 123,7 S_{X} 151,1 I |
| Beispiel 16 | C₈H₁₇-Py-Ph-COO-THP-(CH₂)₄CH₃ | K 75,8 S_{C*} 96,8 S_{A} 97,9 Ch 177,8 I |
| Beispiel 17 | C₁₀H₂₁O-Ph-COO-Ph-COO-THP-(CH₂)₃CH₃ | K 125,3 S_{A} 151,7 I |
| Beispiel 18 | C₁₀H₂₁O-Ph-Ph-COO-THP-(CH₂)₅CH₃ | K 99,8 S_{C*} 89,1 S_{A} 134,6 Ch 136,1 I |
| Beispiel 19 | C₈H₁₇-Py-Ph-COO-THP-(CH₂)₅CH₃ | K 73,2 S_{A} 95,0 Ch 106,1 I |

Strukturen der Beispiele 13 bis 19

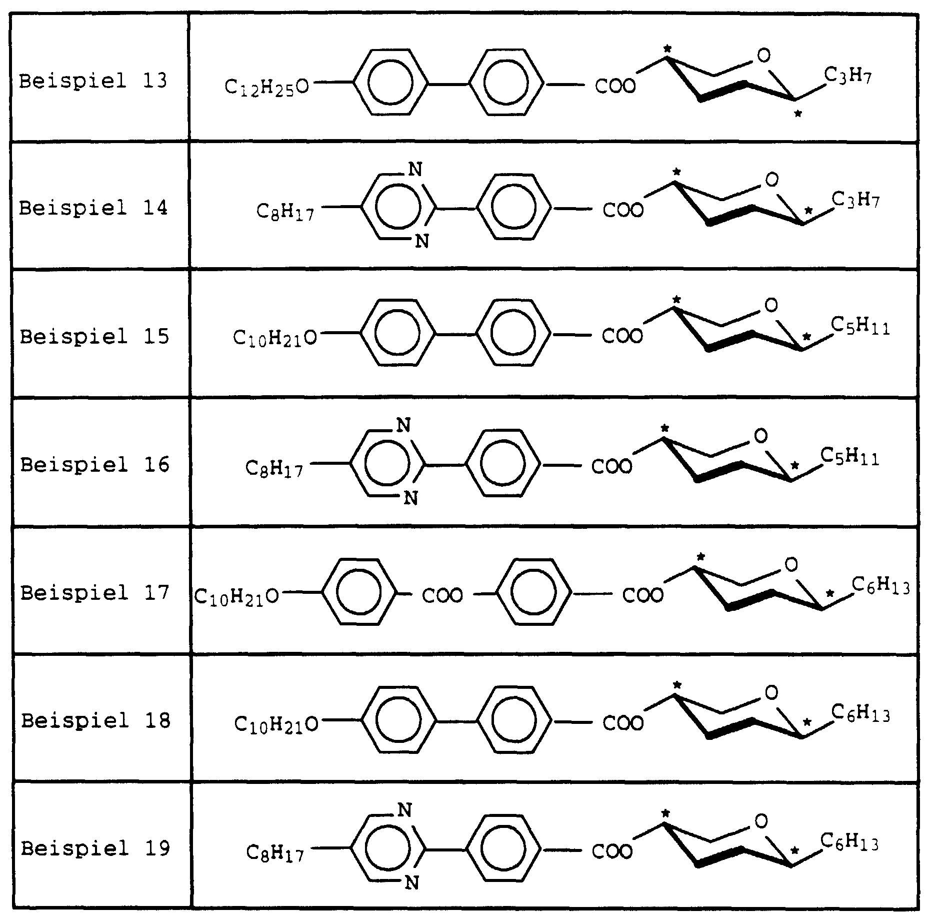

### Beispiel 20

Herstellung von (2S,5S)-5-[trans-4'-(4"-n-Heptylcyclohexyl)-phenyl]-carboxyl-2-(4'-methoxyphenyl)-2H tetrahydropyran 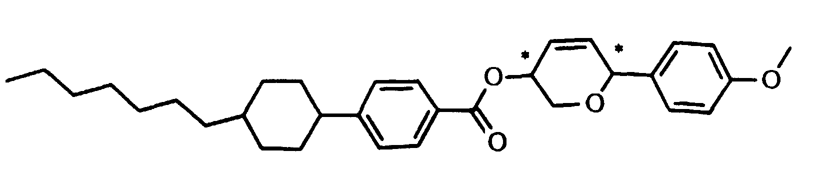
a) Herstellung von (2S,5S)-5-Acetyloxy-2-(4'-methoxyphenyl)-tetrahydropyran
   500 mg (2S,5S)-5-Acetoxy-2-(4'-methoxyphenyl)-2H-5,6-dihydropyran werden in 100 ml eines Gemisches aus Ethanol/Ethylacetat (1/3) gelöst. Diese Lösung wird mit 5 mg Palladium auf Aktivkohle (10%) versetzt und unter Normaldruck einer Wasserstoffatmosphäre hydriert. Die Reaktionskontrolle erfolgt dünnschichtchromatografisch (Laufmittel: Petrolether/Ethylacetat = 9/1). Nach 12 Stunden ist die Reaktion beendet. Vom Katalysator wird abfiltriert und das Lösungsmittel am Rotationsverdampfer abgezogen. Die Aufarbeitung erfolgt säulenchromatografisch (Laufmittel: Petrolether/Ethylacetat = 9/1).
   Ausbeute: 455 mg (91% d. Theorie)
   [α]²⁰_{D} = -64.2° (c=1, Chloroform)
   Schmelzpunkt: 62.2 °C
b) Herstellung von (2S,5S)-5-Hydroxy-2-(4'-methoxyphenyl)-tetrahydropyran
   400 mg (2S,5S)-5-Acetyloxy-2-(4'-methoxyphenyl)-tetrahydropyran werden in 50 ml absolutem Methanol gelöst, bis zur basischen Reaktion mit 0.1 N methanolischer Natriummethanolat-Lösung tropfenweise versetzt und 12 Stunden bei Raumtemperatur gerührt. Anschließend wird mit einem Ionenaustauscher in der H⁺-Form neutralisiert und vom Ionenaustauscher abfiltriert. Nach dem Einengen wird säulenchromatografisch (Laufmittel: Chloroform/Methanol=9/1) /1) gereinigt.
c) Herstellung von (25, 5S)-5- [trans-4- (4"-n-Heptylcyclohexyl)-phenyl]-carboxyl-2-(4'-methoxyphenyl)-2H tetrahydropyran
   29 mg trans-4'-(4"-n-Heptylcyclohexyl)-phenylcarbonsäure werden in der fünffachen Gewichtsmenge Thionylchlorid gelöst und mit einem Tropfen Dimethylformamid versetzt. Dann rührt man bei Raumtemperatur bis eine klare Lösung entstanden ist. Anschließend wird noch für 10 min auf 40°C erwärmt und dann das Lösungsmittel abdestilliert. Das Säurechlorid wird mehrere Stunden unter Ölpumpenvakuum getrocknet.
   20 mg (2S,5S)-5-Hydroxy-2-(4'-methoxyphenyl)-tetrahydropyran und 12 mg Dimethylaminopyridin werden in der fünffachen Gewichtsmenge absolutem Pyridin gelöst und dann zu einer Lösung von 30 mg des Säurechlorides in absolutem Dichlormethan gegeben. Die Mischung rührt über Nacht bei Raumtemperatur und anschließend eine halbe Stunde bei 40°C. Dann wird das Lösungsmittel abdestilliert, der Rückstand dreimal in Toluol aufgenommen und wieder zur Trockne eingedampft. Die Reinigung des so erhaltenen Produktes erfolgt säulenchromatografisch mit Dichlormethan als Laufmittel.
   Ausbeute: 14.6 g (31% d. Theorie)
   [α]²⁰_{D} = 32.4 (c=1, Chloroform)
   Phasenverhalten: K 100.1 Ch 221.0 I

Analog Beispiel 20 wurden die Verbindungen der Beispiele 21 bis 30 hergestellt:

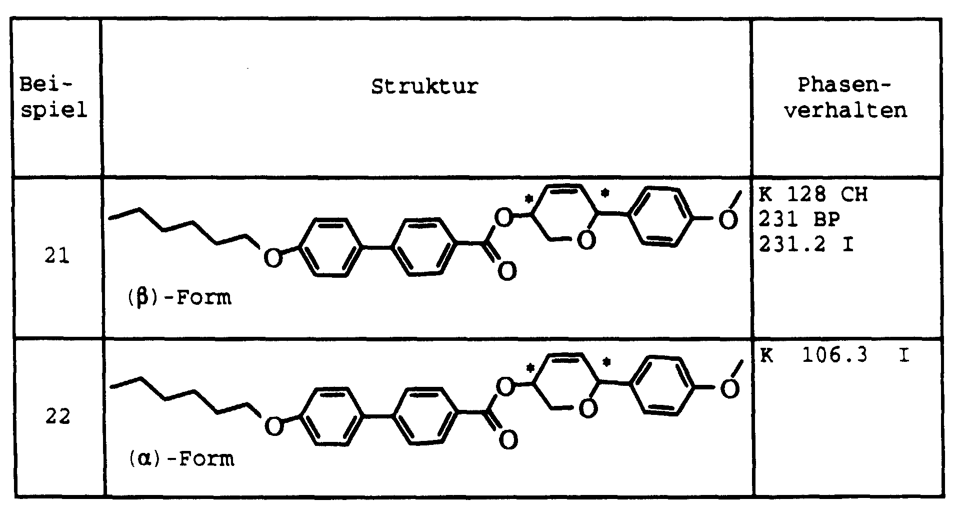

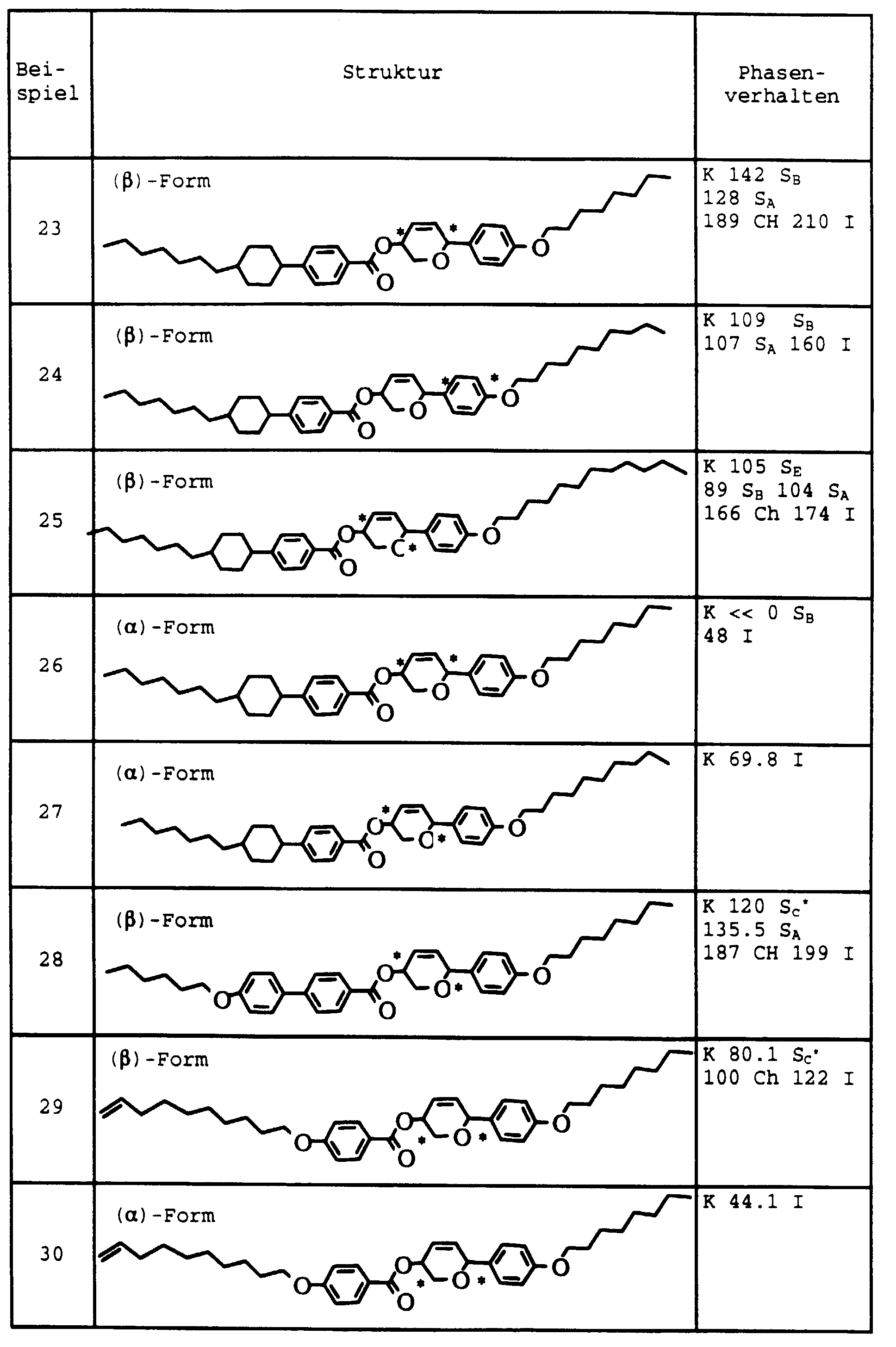

## Patentansprüche

1. Verbindungen der Formel I 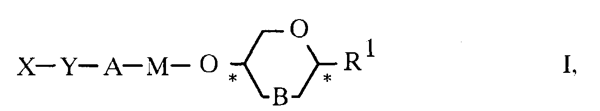 in der bedeuten
R¹ gegebenenfalls durch Fluor, Chlor, Brom, Cyan oder Hydroxy substituiertes und gegebenenfalls durch O, S, NH, N(CH₃), COO oder OCO unterbrochenes C₁- bis C₃₀-Alkyl, gegebenenfalls durch Fluor, Chlor, Brom, Cyan, C₁- bis C₂₀-Alkyl, C₁- bis C₂₀-Alkoxy oder C₁- bis C₂₀-Alkoxycarbonyl substituiertes Phenyl oder Fluor, Chlor, Brom oder Cyan,
B eine Einfach- oder Doppelbindung,
X eine polymerisierbare Gruppe oder Wasserstoff oder Hydroxy,
Y eine direkte Bindung oder O, OCO, COO oder S,
A ein gegebenenfalls durch Fluor, Chlor, Brom, Cyan oder Hydroxy substituierter C₁- bis C₃₀-Alkylrest, bei dem gegebenenfalls jedes dritte C-Atom durch O, S, NH, N(CH₃), OCO oder COO ersetzt sein kann, und
M eine mesogene Gruppe der Formel 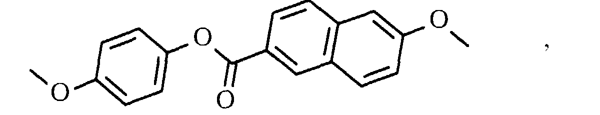 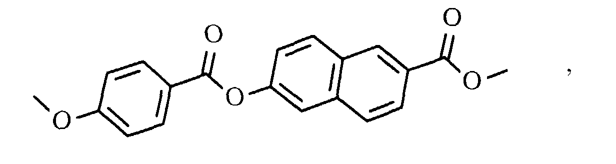 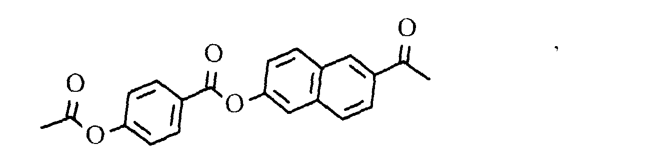 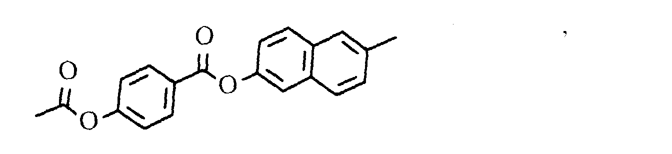 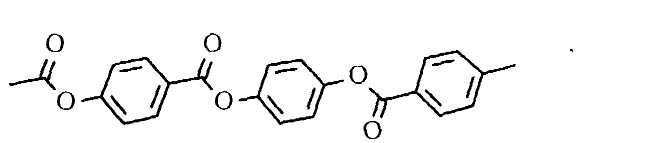 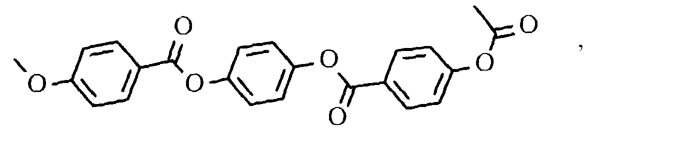 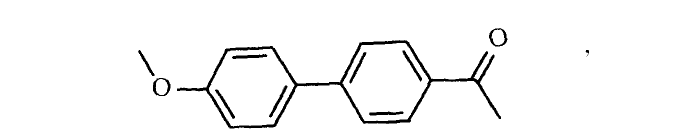 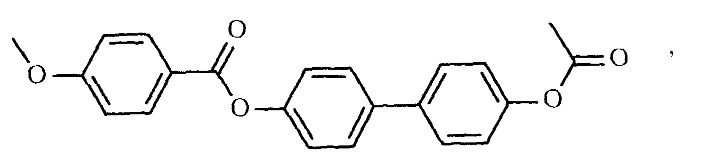 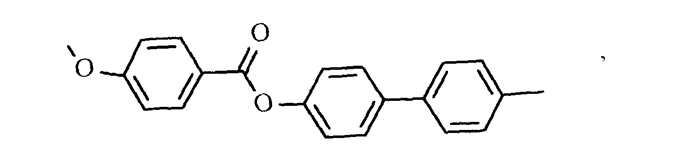 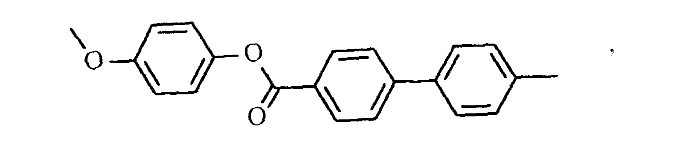 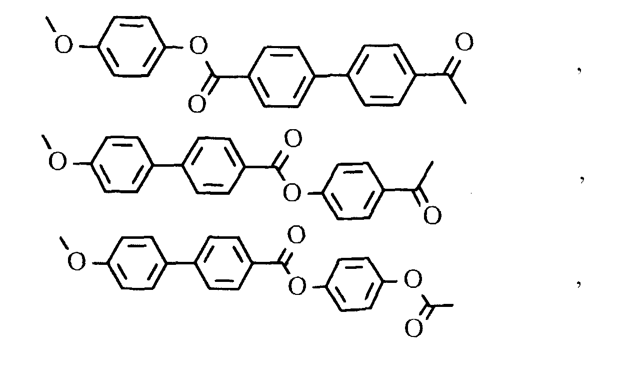 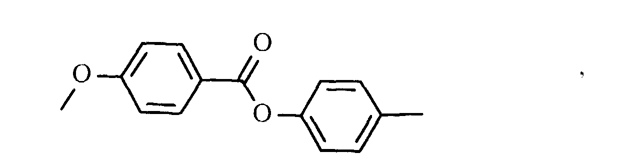 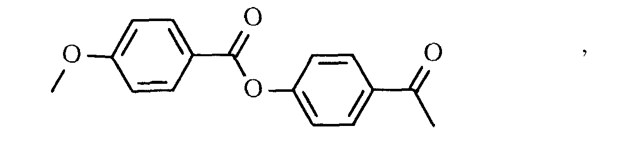 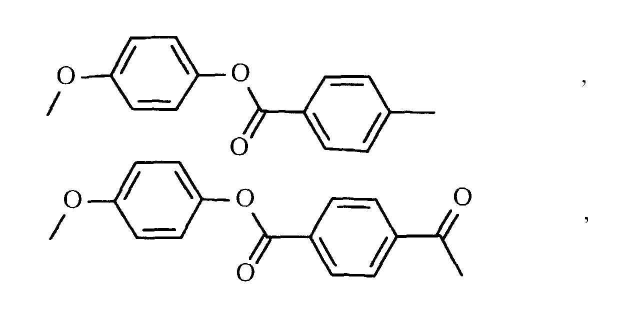 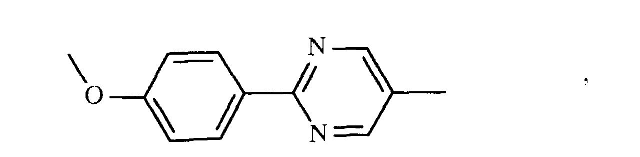 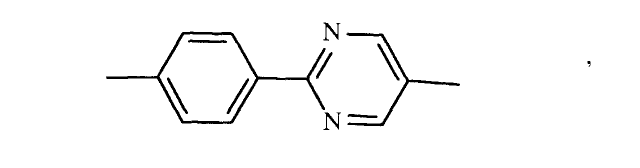 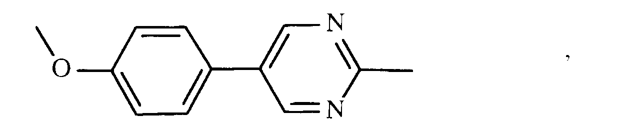 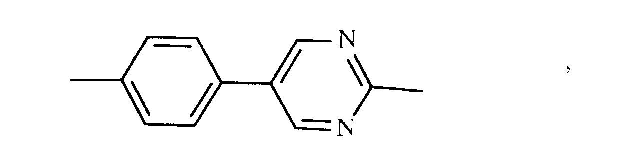 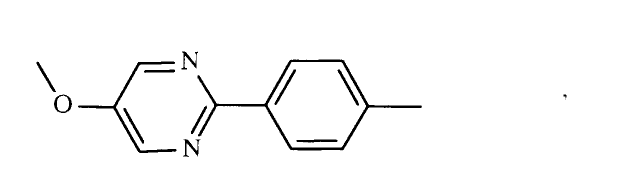 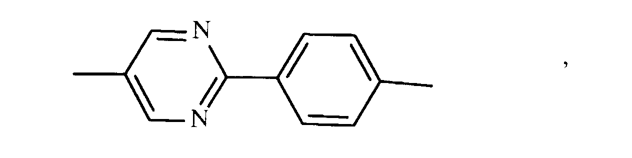 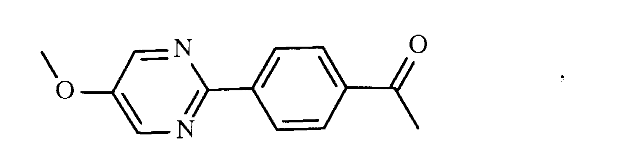 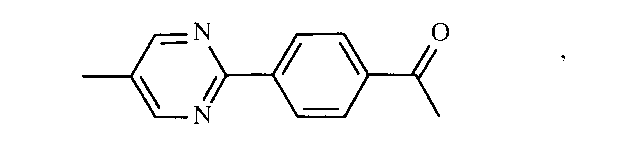 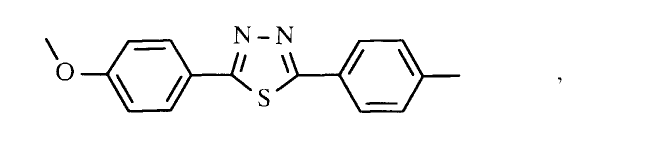 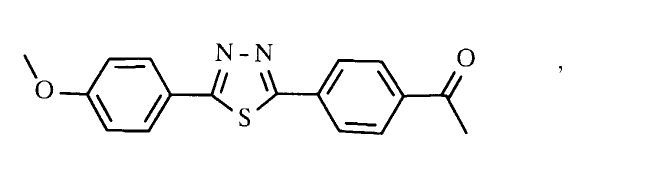 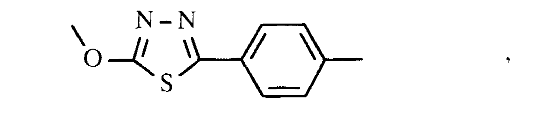 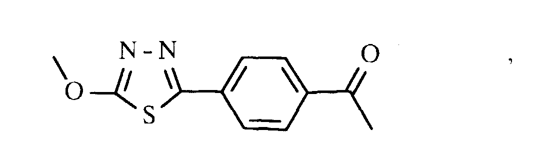 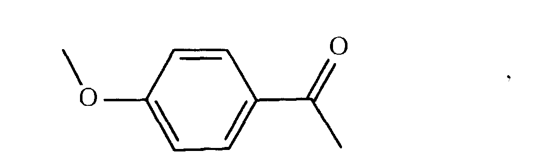 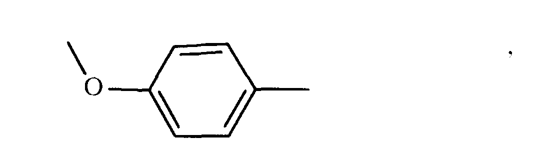 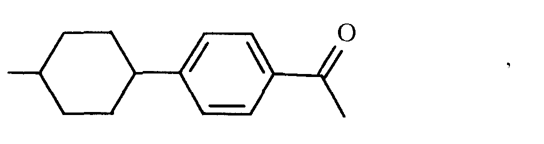 oder

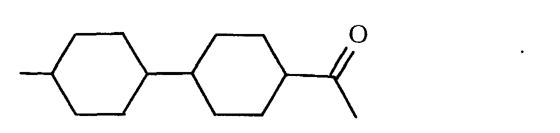

2. Verbindungen gemäß Anspruch 1 mit B als Einfachbindung.

3. Verbindungen gemäß Anspruch 1 mit B als Doppelbindung.

4. Verwendung der Verbindungen gemäß Anspruch 1 mit einer polymerisierbaren Gruppe X zur Herstellung polymerer chiraler Verbindungen.

5. Verwendung der Verbindungen gemäß Anspruch 1 mit X=OH oder X=Vinyl zur Herstellung von chiralen Oligomesogenen.

6. Verwendung der Verbindungen gemäß Anspruch 1 als Dotierstoff in flüssigkristallinen Medien.

7. Verwendung von flüssigkristallinen Medien enthaltend Verbindungen gemäß Anspruch 1 in der Displaytechnologie, in optischen, elektronischen sowie opto-elektronischen Speichermedien, der elektronischen Datenverarbeitung und Vervielfältigung oder der Elektrophotografie sowie in Licht reflektierenden Schichten.

## Claims

1. A compound of the formula I 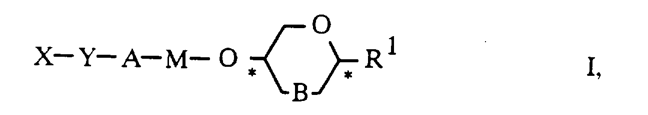 where
R¹ is C₁-C₃₀-alkyl which is unsubstituted or substituted by fluorine, chlorine, bromine, cyano or hydroxyl and may be interrupted by O, S, NH, N(CH₃), COO or OCO, or is phenyl which is unsubstituted or substituted by fluorine, chlorine, bromine, cyano, C₁-C₂₀-alkyl, C₁-C₂₀-alkoxy or C₁-C₂₀-alkoxycarbonyl, or is fluorine, chlorine, bromine or cyano,
B is a single or double bond,
X is a polymerizable group or hydrogen or hydroxyl,
Y is a direct linkage or O, OCO, COO or S,
A is a C₁-C₃₀-alkyl radical which is unsubstituted or substituted by fluorine, chlorine, bromine, cyano or hydroxyl and in which each third carbon atom can be replaced by O, S, NH, N(CH₃), OCO or COO, and
M is a mesogenic group of the formula 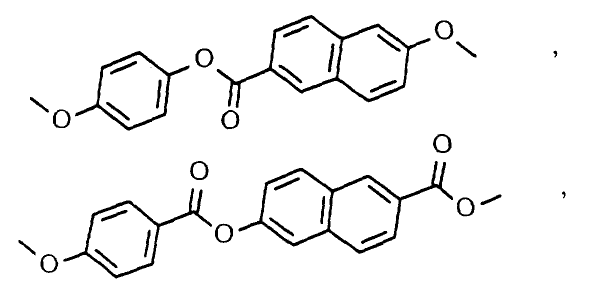 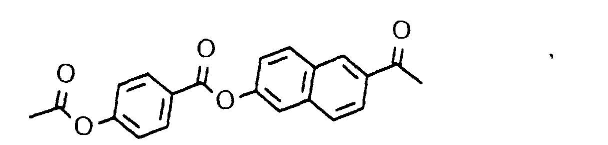 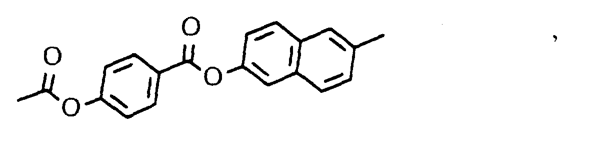 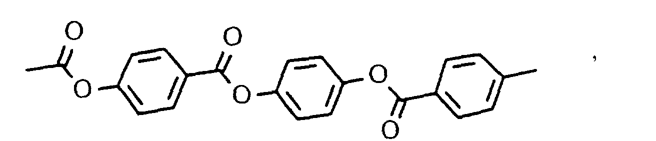 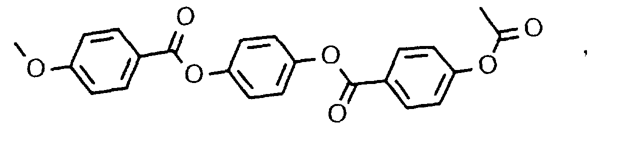 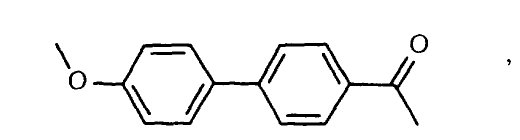 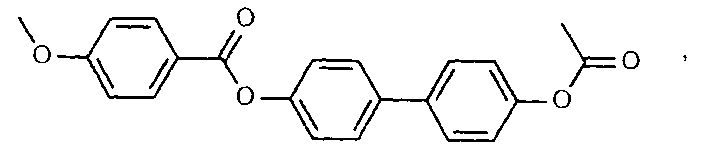 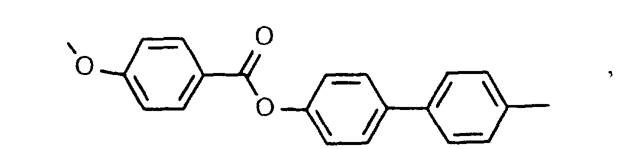 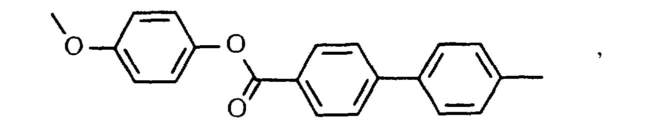 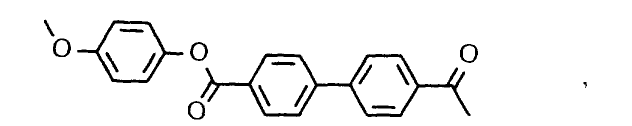 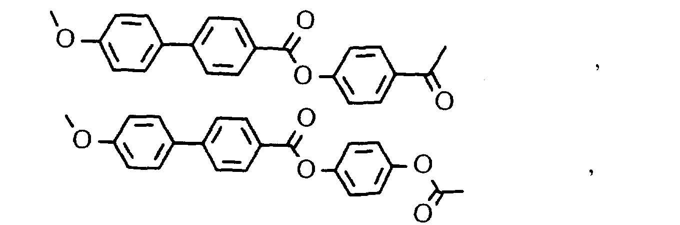 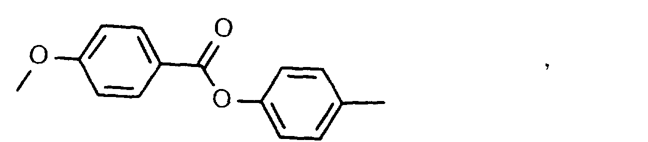 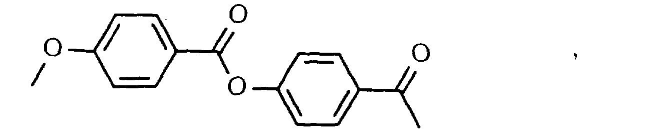 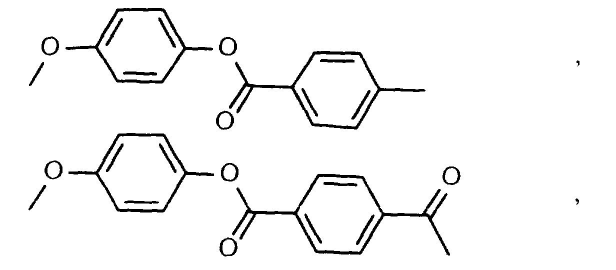 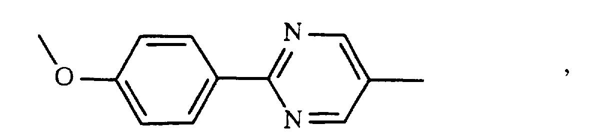 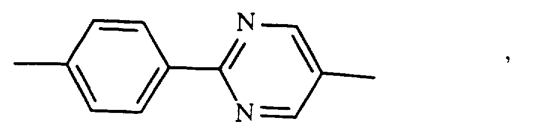 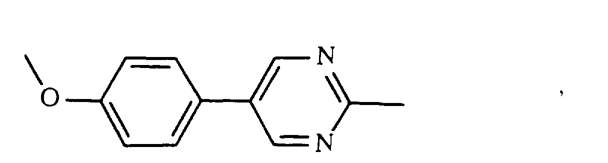 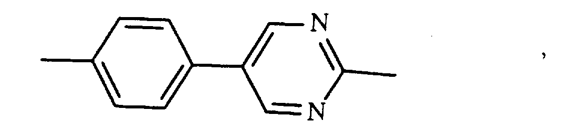 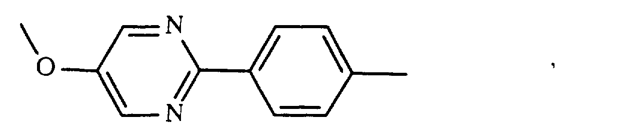 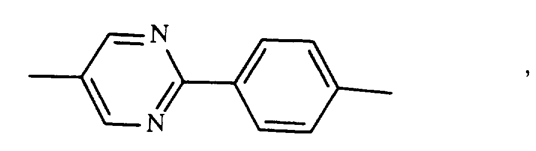 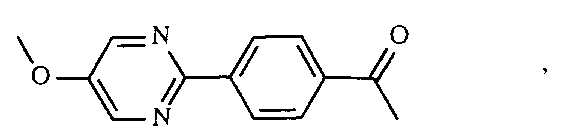 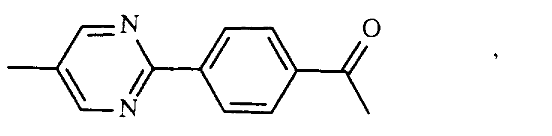 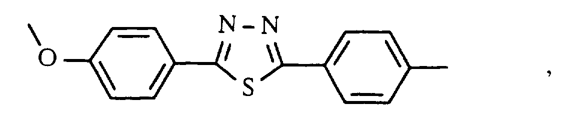 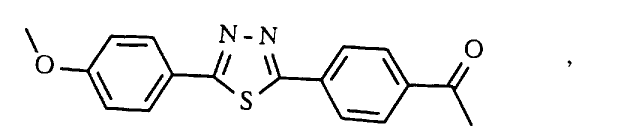 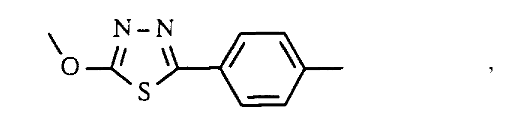 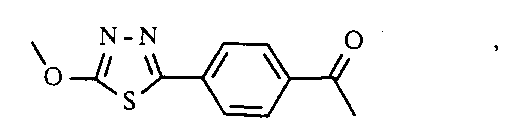 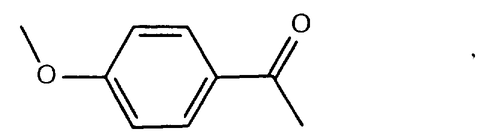 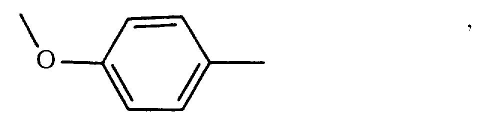 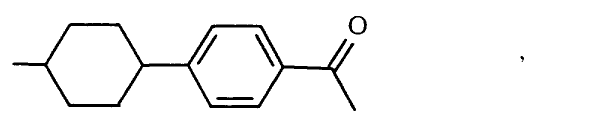 or

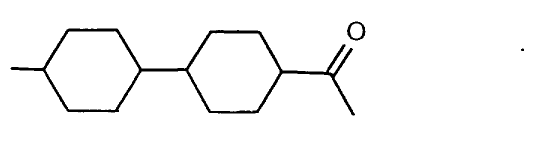

2. A compound as claimed in claim 1 with B as a single bond.

3. A compound as claimed in claim 1 with B as a double bond.

4. The use of a compound as claimed in claim 1 with a polymerizable group X for preparing polymeric chiral compounds.

5. The use of a compound as claimed in claim 1 with X = OH or X = vinyl for preparing chiral oligomesogens.

6. The use of a compound as claimed in claim 1 as dopant in liquid-crystalline media.

7. The use of liquid-crystalline media containing compounds as claimed in claim 1 in display technology, in optical, electronic and optoelectronic storage media, electronic data processing and reproduction or electrophotography and in light-reflecting layers.

## Revendications

1. Composés de formule I 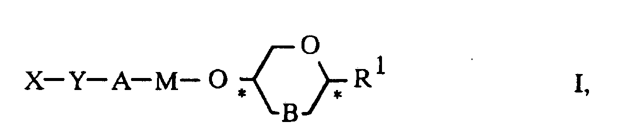 dans laquelle
R¹ représente un groupement alkyle en C₁-C₃₀ éventuellement substitué par un atome de fluor, de chlore, de brome, un groupement cyano ou hydroxy, et éventuellement interrompu par O, S, NH, N(CH₃), COO ou OCO, un groupement phényle éventuellement substitué par un atome de fluor, de chlore, de brome, un groupement cyano, alkyle en C₁-C₂₀, alcoxy en C₁-C₂₀ ou alcoxycarbonyle en C₁-C₂₀, ou un atome de fluor, de chlore,de brome ou un groupement cyano,
B représente une liaison simple ou double,
X représente un groupement polymérisable ou un atome d'hydrogène ou un groupement hydroxy,
Y représente une liaison directe ou O, OCO, COO ou S,
A représente un reste alkyle en C₁-C₃₀ éventuellement substitué par un atome de fluor, de chlore, de brome, un groupement cyano ou hydroxy, dans lequel, éventuellement, tous les trois atomes de C, un atome de C peut être remplacé par O, S, NH, N(CH₃), OCO ou COO, et
M représente un groupement mésogène de formule 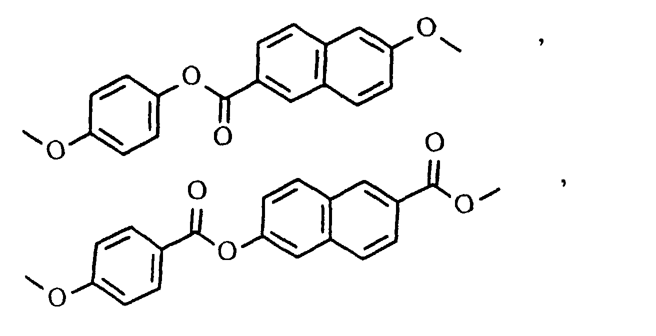 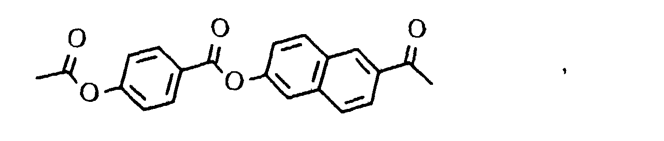 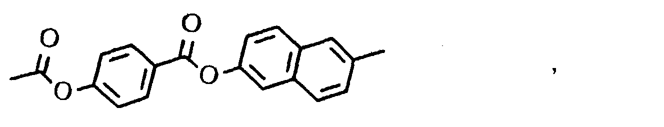 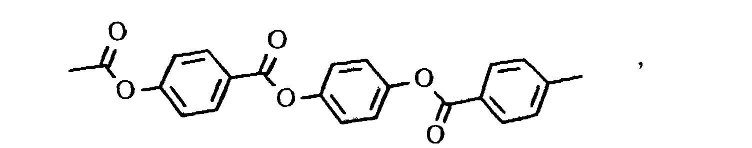 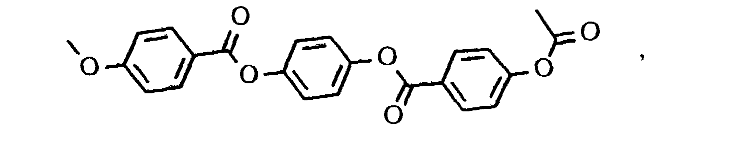 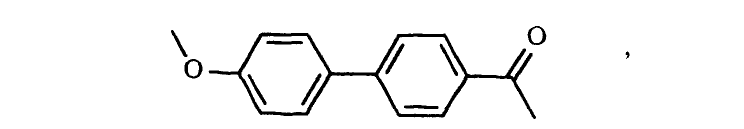 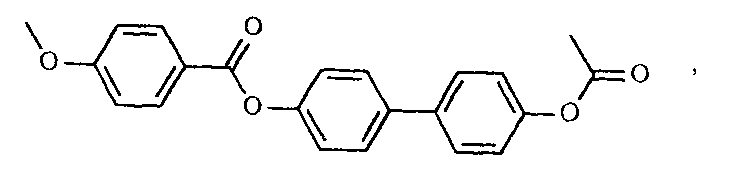 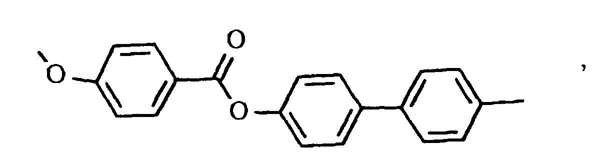 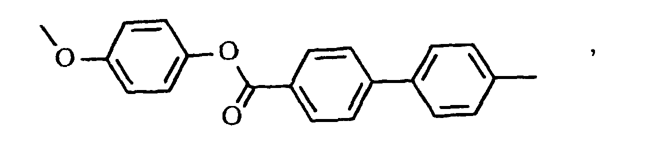 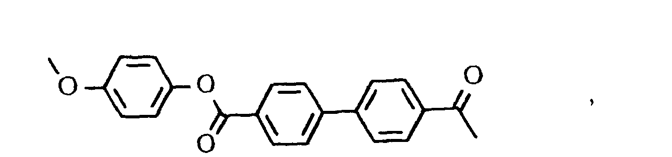 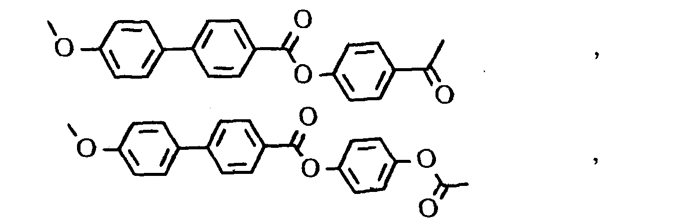 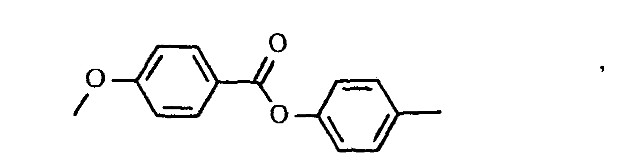 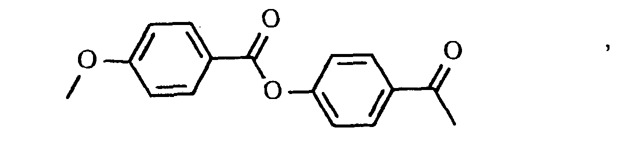 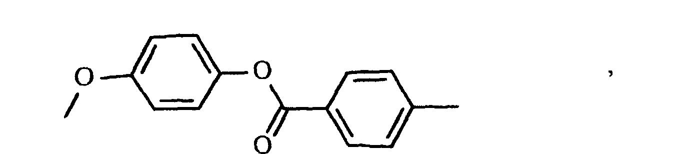 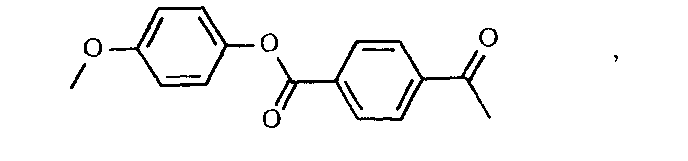 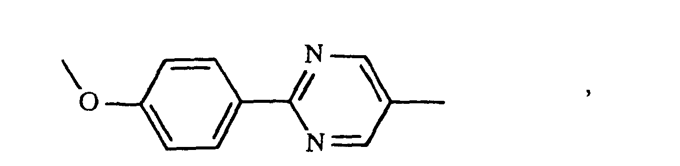 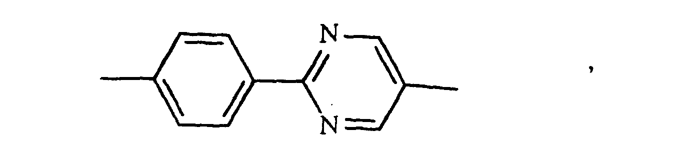 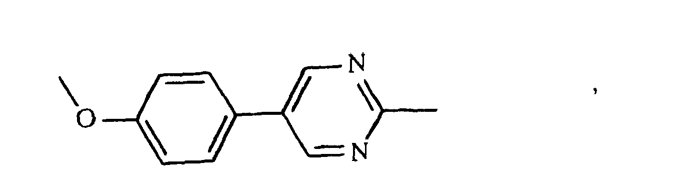 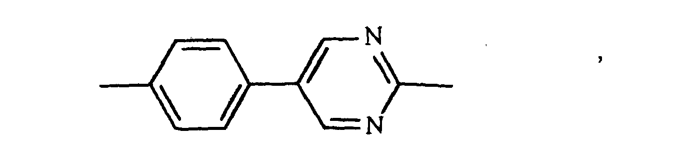 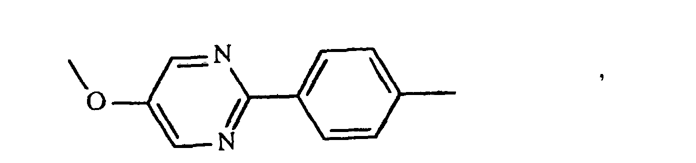 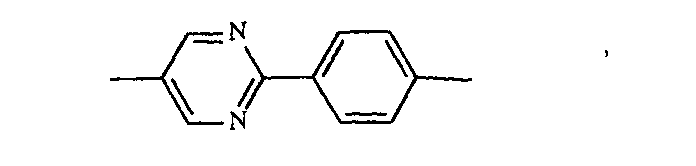 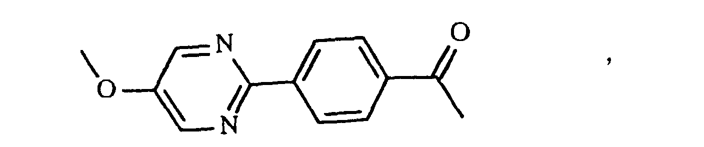 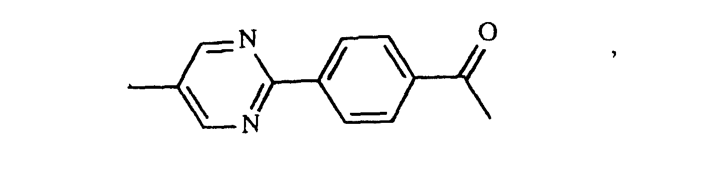 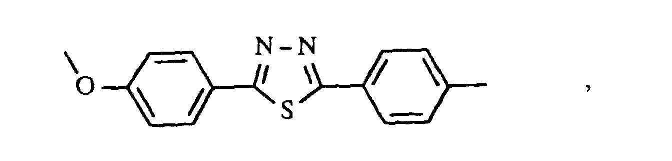 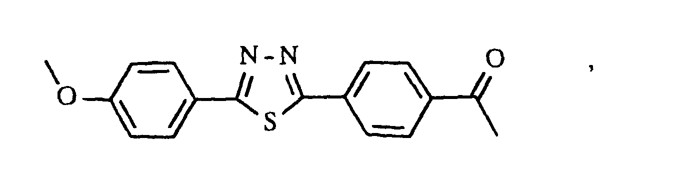 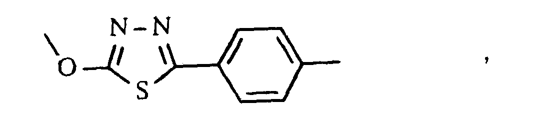 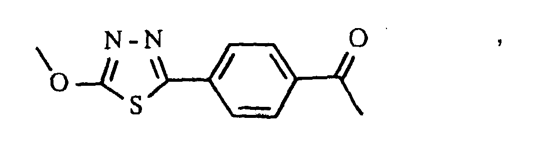 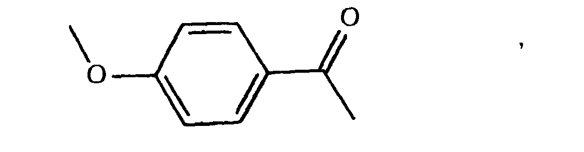 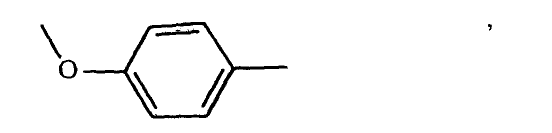 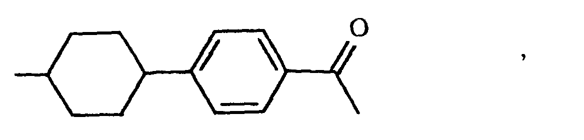 ou

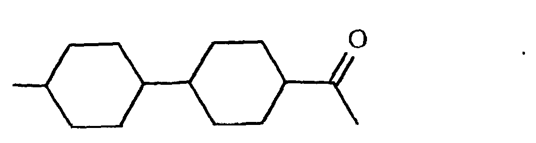

2. Composés selon la revendication 1, pour lesquels B représente une simple liaison.

3. Composés selon la revendication 1, pour lesquels B représente une double liaison.

4. Utilisation de composés selon la revendication 1, ayant un groupement X polymérisable, pour la préparation de composés chiraux polymères.

5. Utilisation de composés selon la revendication 1, pour lesquels X=OH ou X=vinyle, pour la préparation d'oligomésogènes chiraux.

6. Utilisation de composés selon la revendication 1, en tant qu'agent dopant dans des milieux cristaux liquides.

7. Utilisation de milieux cristaux liquides contenant des composés selon la revendication 1, dans la technologie de l'affichage, dans des moyens de stockage optiques, électroniques ou opto-électroniques, dans le traitement de données et la reproduction électronique ou dans l'électrophotographie ainsi que dans les couches réfléchissant la lumière.
